# EUROPÄISCHE PATENTANMELDUNG

(11) **EP 4 614 132 A1**
(43) Veröffentlichungstag der Anmeldung: **10.09.2025**
(21) Anmeldenummer: 24161584.8
(22) Anmeldetag: 05.03.2024
(51) Int. Cl.: G01N 21/00

(54) **PRÜFVORRICHTUNG ZUR AUTOMATISIERTEN SCHWEISSNAHTPRÜFUNG UND VERFAHREN ZUR SCHWEISSNAHTPRÜFUNG**

(71) Anmelder: EEW Special Pipe Constructions GmbH, 18147 Rostock (DE)
(72) Erfinder: SCHWARZ, Matthias, 18233 Neubukow (DE)
(74) Vertreter: Bardehle Pagenberg Partnerschaft mbB Patentanwälte Rechtsanwälte

(57) **Zusammenfassung**

Gegenstand der Erfindung ist eine mobile Prüfvorrichtung (1) zur Prüfung von Rundschweißnähten eines Großrohrs mit mindestens einem verfahrbaren Prüfwagen (2) als mobile Basiseinheit, wobei der Prüfwagen mindestens drei Räder hat, die an der Basiseinheit montiert sind, mindestens eine Messeinheit (3) zur Prüfung der Schweißnaht, mindestens eine Sende- und Empfangseinheit (4), mindestens eine Steuereinheit (5) , und ggf. eine Antriebseinheit (6) . Die Prüfvorrichtung weist außerdem eine Markiereinheit auf. Weiterhin ist Gegenstand der Erfindung ein zum Prüfen einer Rundschweißnaht an einem Großrohr. Die Geschwindigkeit der Prüfung einer Schweißnaht ist gegenüber der visuellen Prüfung deutlich erhöht. Der menschliche Einfluss bei der Schweißnahtprüfung und die subjektive Beurteilung wird durch die Verwendung der Prüfvorrichtung und das Verfahren eliminiert.

## Beschreibung

Die Erfindung betrifft eine mobile Prüfvorrichtung zur Prüfung von Rundschweißnähten eines Großrohrs gemäß dem Oberbegriff von Anspruch 1 und ein Verfahren zur Schweißnahtprüfung gemäß Anspruch 14.

Ein wichtiger Punkt für die Qualität von geschweißten Rohren aus mehreren Rohschüssen ist die Qualität der Rundschweißnähte. Das Schweißen der Rundnähte erfolgt üblicherweise mit Unterpulver-Schweißen. Speziell für Großrohr mit einem Durchmesser von mehr als 6m, die im Bereich Offshore-Fundamente eingesetzt werden, ist eine hohe Prüfabdeckung (100%) aller Nähte gefordert, um die Dauerfestigkeit über mindestens 20 Jahre zu gewährleisten. Gemäß der anzuwendenden Norm DIN ISO 5817 erfolgt eine visuelle Prüfung mit dem Auge. Die Norm beschreibt dabei die optisch verschiedenen Nahtformen und die sich daraus ergebenden Fehlstellen. Wenn bei der visuellen Prüfung Fehstellen oder Mängel festgestellt werden, werden diese händisch markiert, damit eine Nachbearbeitung erfolgen kann. zudem werden die Fehlstellen in einem Prüfprotokoll vermerkt.

Der Aufwand für die Schweißnahtprüfung ist daher sehr hoch ist, da ausreichend Personal für eine vollständige Prüfung aller Schweißnähte eingesetzt werden muss. Bei der visuellen Prüfung stellen sich mehrere Probleme. Das menschliche Auge ermüdet während des Arbeitstages und wird bei der Prüfung ungenauer. Durch die visuelle Prüfung ist immer eine subjektive Bewertung gegeben, da jeder Prüfer unterschiedlich kritisch prüft. Es ist zudem kaum möglich die visuelle Prüfung zu dokumentieren, da Fotos nur ein zweidimensionales Bild wiedergeben, jedoch nicht die dreidimensionale Beschaffenheit abbilden und Prüfprotokolle nur einen unvollständigen Bericht über den Zustand der Naht geben.

Es gibt deshalb ein Bestreben, die Prüfung von Schweißnähten maschinell durchzuführen. DE 10134696 C1 beschreibt Vorrichtung zum Ultraschallprüfen der Schweißnaht längsnahtgeschweißter Rohre auf Längs- und Querfehler. Die Vorrichtung weist für die Prüfung zwei schwenkbar aufgehängten, auf der Oberfläche des Rohres rechts und links neben der Schweißnaht verschiebbaren Prüfwagen für die Längsfehler-Prüfung und einem schwenkbar aufgehängten, mittig zur Schweißnaht ausgerichteten, auf der Oberfläche des Rohres verschiebbaren Prüfwagen für die Querfehlerprüfung. Die Vorrichtung hat einen relativ komplexen Aufbau und benötigt mehrere Prüfungswagen für die verschiedenen Prüfungsaufgaben.

Aufgabe der vorliegenden Erfindung ist es die vorstehenden Nachteile zu überwinden und insbesondere den subjektiven Anteil bei der Bewertung zu reduzieren, die Geschwindigkeit der Prüfung zu erhöhen und die mit der Prüfung beauftragten Personen zu entlasten.

Die Aufgabe wird erfindungsgemäß gelöst durch eine mobile Prüfvorrichtung zur Prüfung von Rundschweißnäht eines Großrohrs gemäß Anspruch 1. Weiterhin wird die Aufgabe gelöst durch ein Verfahren zum Prüfen einer Rundschweißnaht an einem Großrohr gemäß Anspruch 12.

Weitere Ausführungsformen sind Gegenstand der Unteransprüche oder nachfolgend beschreiben.

Die erfindungsgemäße mobile Prüfvorrichtung zur Prüfung von Rundschweißnähten eines Großrohrs weist mindestens die folgenden Komponenten auf
- einen verfahrbaren Prüfwagen als mobile Basiseinheit, wobei der Prüfwagen mindestens drei Räder hat, die an der Basiseinheit montiert sind,
- mindestens eine Messeinheit zur Prüfung der Schweißnaht,
- mindestens eine Sende- und Empfangseinheit,
- mindestens eine Steuereinheit, und
- ggf. eine Antriebseinheit, die den Prüfwagen antreibt, beispielweise mindestens ein Elektromotor.

Die Messeinheit, die Sende- und Empfangseinheit, ggf. die Antriebseinheit und ggf. die Steuereinheit sind an der mobilen Basiseinheit, d.h. an dem Prüfwagen, befestigt und werden von dieser getragen. Die Messeinheit ist eine Lasereinheit, mit der die Beschaffenheit der Schweißnaht gemessen wird. Die Beschaffenheit der Schweißnaht umfasst das Höhenprofil der Schweißnaht und die Breite der Schweißnaht. Die Prüfvorrichtung weist mindestens eine Markiereinheit auf, die an der mobilen Basiseinheit befestigt ist. Mit der Markiereinheit werden Fehlstellen oder stellen, die einer Nacharbeitung oder Begutachtung bedürfen, markiert.

Bevorzugt ist die Messeinheit eine Lasereinheit, die mindestens einen Laserkopf mit einem Emitter und mit einem Detektor hat, wobei der Emitter mindestens einen Laserstrahl aussendet und der Detektor den reflektierten Laserstrahl auffängt. Die Lasereinheit weist bevorzugt einen Lichtlaser auf.

Der Laserkopf ist bevorzugt über eine Aufnahmevorrichtung mit einer Einstellvorrichtung an der Basiseinheit befestigt, wobei mit der Einstellvorrichtung der Abstand zwischen Rohroberfläche und Laser eingestellt wird. Die Einstellvorrichtung ist beispielsweise eine Stellschraube oder eine andere Stellvorrichtung. Mit der Einstellvorrichtung wird der Abstand zwischen Lasereinheit und Rohroberfläche eingestellt und so an den Innendurchmesser und Außendurchmesser des zu prüfenden Rohrs angepasst.

Der Prüfwagen kann eine Lenkeinheit aufweisen, wobei die Lenkeinheit mit den Rädern verbunden ist und die Lenkeinheit die Räder lenkt.

Die Lenkeinheit kann mit der Steuereinheit verbunden sein, wobei die Steuereinheit die Lenkeinheit steuert und die Steuereinheit die Signale über eine manuelle Steuerung z.B. über eine Steuerung mit einem Joystick oder Steuerknüppel oder über eine automatisierte Steuerung mit einer Software erhält.

Wenn der Prüfwagen mindestens eine Antriebseinheit aufweist, die den Prüfwagen antreibt, beispielweise mindestens ein Elektromotor, kann die Antriebseinheit mit mindestens einer Steuereinheit verbunden sein. Die Steuereinheit kann die Vorwärts- oder Rückwärtsbewegung des Prüfwagens und dessen Geschwindigkeit steuern.

Die Prüfvorrichtung kann mindestens eine Energieversorgung, bevorzugt einen Akkumulator, deine Batterie oder einen Netzanschluss aufweisen. Die Energieversorgung kann als interner oder externer Akkumulator oder Akkupalet ausgebildet sein und die Antriebseinheit, die Pumpe, die Steuereinheit und alle elektrischen oder elektronischen Systeme der Prüfvorrichtung mit Strom versorgen.

Bei einer automatisierten Steuerung des Prüfwagens kann die Steuerung über eine Software erfolgen, die eine programmierte Nahterkennung hat. Die Messeinheit erkennt die Lage der Naht und sendet über die Sendeeinheit die Information an die Steuereinheit. Die Steuereinheit bestimmt hieraus die relative Position des Prüfwagens zur Naht. Bei Erreichung eines vorgegebenen Schwellenwertes sendet die Steuereinheit ein Signal an die Lenkeinheit und die Lenkeinheit korrigiert die Lenkung nach links oder rechts, um den Prüfwagen relativ zur Naht auszurichten.

Es ist vorteilhaft, wenn die Prüfvorrichtung eine Nahterkennung hat und der Prüfwagen basierend auf den Daten der Nahterkennung gesteuert wird.

Eine Steuereinheit kann als Chip, als Softwareeinheit oder als Recheneinheit ausgebildet sein und in einem Gerätegehäuse mit einem Filtersystem gekapselt sein. Das Gehäuseinnere ist bevorzugt mit einem Überdruck beaufschlagt.

Durch die Kapselung der Steuereinheit in einem separaten Gerätegehäuse mit ggf. einem durch Filtersystem am Gehäuse und ggf. Überdruck im Gehäuse wird der Eintrag von Metallstaub oder anderen Verunreinigungen vermieden, welche auf lange Sicht zur Verunreinigung der Steuereinheit führen würde. Durch die Verunreinigungen besteht eine Kurzschlussgefahr der Steuereinheit die mit Hilfe des Gehäuses vermieden wird.

Eine Steuereinheit kann im Prüfwagen oder extern angeordnet sein, z.B. in einem externen Behälter. Neben einer Steuereinheit für mindestens einen Markiereinheit, kann die Prüfvorrichtung auch Steuereinheiten für den Prüfwagen, z.B. für eine Lenkeinheit und/oder eine Antriebseinheit des Prüfwagen, und eine Steuereinheit für die Messeinheit aufweisen.

Die Prüfvorrichtung kann eine Auswerteeinheit aufweisen, die die von der Messeinheit erhaltenen Daten, wie z.B. die gemessenen Werte, auswertet, wobei die Auswerteeinheit bevorzugt eine Speichereinheit aufweist, die die von der Messeinheit erhaltenen Daten und die von der Auswerteinheit erzeugten Daten speichert.

In einer Ausführungsform weist die Markiereinheit mindestens einen Markierkopf, z.B. einen Tintenkopf, mindestens einen Vorratsbehälter für Farbe und eine Farbleitung zwischen Markierkopf und Vorratsbehälter auf. Durch den Aufbau der Markiereinheit auf dem Prüfwagen kann die Farbleitung so kurz wie möglich gehalten werden, damit Farbe nur über eine kurze Strecke transportiert werden muss. Die Farbleitung oder Farbleitungen bilden ein Farbführungssystem mit dem Farbe, wie z.B. Tinte, aus dem Vorratsbehälter, z.B. einem Farbbeutel, zum Markierkopf geführt wird.

Die Markiereinheit kann eine Halterung aufweisen und mit der Halterung an einer Aufnahmevorrichtung ggf. mit einer Einstellvorrichtung an der Basiseinheit befestigt sein. Über eine Einstellvorrichtung kann der Abstand zwischen Rohroberfläche und Markierkopf eingestellt wird. Die Einstellvorrichtung ist beispielsweise eine Stellschraube oder eine andere Stellvorrichtung. Mit der Einstellvorrichtung ist der Abstand zur Rohroberfläche einstellbar und so an den Innendurchmesser und Außendurchmesser des zu prüfenden Rohrs anpassbar.

Die Markierköpfe der Markiereinheit sind bevorzugt mit der Steuereinheit verbunden und die Steuereinheit steuert die Markierköpfe. Bei einem entsprechenden Signal gegeben die Markierköpfe Farbe ab und markieren so eine Stelle auf der Rohroberfläche bzw. auf der Naht. Durch das Signal kann auch die Dauer und Menge der Farbenabgabe gesteuert werden. Die Markierköpfe können feststehenden oder beweglich ausgebildet sein. Bei beweglichen Markierköpfen kann die Steuereinheit auch Bewegung und Positionierung der Markierköpfe steuern.

Die Ansteuerung der Markierköpfe über die Steuereinheit kann basierend auf einer Echtzeitauswertung der Daten der Messeinheit erfolgen. Die Markiereinheit kann dabei z.B. mit dem Markierkopf an der linken Nahtflanke, der rechten Nahtflanke oder der Mitte der Schweißnaht positioniert sein. Die Messeinheit kann entsprechend die Schweißnaht an der linken Nahtflanke, der rechten Nahtflanke oder die Mitte der Schweißnaht vermessen.

Die Markiereinheit kann eine druckgesteuerte Schlauchpumpe aufweisen, die mit dem Vorratsbehälter verbunden ist, wobei die Schlauchpumpe die Farbe aus dem Vorratsbehälter zum Markierkopf pumpt. Die Schlaupumpe weist bevorzugt einen Membrandruckschalter auf. Die Schlauchpumpe wird bevorzugt über 24V betrieben wird. Die Markiereinheit benötigt bevorzugt 1 bar Arbeitsdruck. Da Druckluftleitungen ein hohes Gewicht haben, bietet die Erzielung des Überdrucks von 1bar in der Farbleitung über die druckgesteuerte Schlauchpumpe den Vorteil, dass die Konstruktion ein geringeres Gewicht hat und so leichter an der Rohroberfläche haftet. Der Membrandruckschalter öffnet und schließt einen Kontakt, um den Leitungsdruck über die Schlauchpumpe aufrechtzuerhalten.

Der Vorratsbehälter der Markiereinheit kann eine Befestigung mit Lager aufweisen und mit der Befestigung drehbar auf der Basiseinheit gelagert sein. Durch die drehbare Lagerung wird in einer Überkopfsituation der Farbenfüllstand ausgeglichen, so dass Farbe gefördert werden kann.

Die Räder sind bevorzugt als magnetische Räder ausgebildet.

Die Basiseinheit weist bevorzugt mindestens zwei Sätze von Rädern auf, wobei jeder Radsatz zwei Räder umfasst und die Räder drehbar an einer Achse gelagert sind und wobei jeweils ein Rad des Radsatzes auf der rechten Seite der Basiseinheit angeordnet ist und wobei jeweils ein Rad des Radsatzes auf der linken Seite der Basiseinheit angeordnet ist.

Gegenstand der Erfindung ist weiterhin ein Verfahren zum Prüfen einer Rundschweißnaht an einem Rohr. Das erfindungsgemäße Verfahren umfasst die Schritte
a) Bereitstellen eines Rohrs mit Rundschweißnaht ggf. auf einer Drehvorrichtung,
b) Bereitstellen einer Prüfvorrichtung gemäß einer der Patentansprüche 1 bis 11,
c) Aufsetzen des Prüfwagens der Prüfvorrichtung auf die Rohroberfläche im Bereich der Schweißnaht,
d) Fahren des Prüfwagens der Prüfvorrichtung entlang der Schweißnaht und gleichzeitige Messen der Beschaffenheit der Schweißnaht mit der Messeinheit durch Aussenden eines Laserstrahls mit mindestens einem Emitter und Auffangen des reflektierten Strahls mit einem Detektor,
e) Senden der Messwerte (Istwerte) an eine Steuereinheit und Vergleichen eines Messwerts mit einem auf der Steuereinheit gespeicherten Sollwert,
f) bei einer Abweichung eines Messwerts vom Sollwert, Senden eines Signals an die Markiereinheit, Aktivieren der Markiereinheit und Markieren der Rohroberfläche durch die Markiereinheit.

Die in Schritt e) gesendeten Messwerte können in einem weiteren Schritt an eine Speichereinheit gesendet und auf der Speichereinheit gespeichert werden.

Das Rohr kann in Schritt a) auf einer Drehvorrichtung bereit gestellt werden. In diesem Fall wird das Rohr mit der Drehvorrichtung um seine Längsachse gegenläufig zur Fahrtrichtung des Prüfwagens gedreht, während der Prüfwagen gemäß Schritt d) entlang der Schweißnaht fährt.

Das Rohr hat einen Durchmesser von mindestens 6 m, bevorzugt einen Durchmesser von mindestens 8 m oder 10 m.

Das Verfahren zum Prüfen einer Rundschweißnaht eines Großrohrs ermöglicht somit eine automatisierte Prüfung der Schweißnaht. Der Prüfwagen mit Rädern wird auf das Rohr aufgesetzt, wobei die Räder bevorzugt magnetisch sind, so dass der Prüfwagen an der Rohroberfläche haftet. Der Prüfwagen trägt als Messeinheit bevorzugt einen Linienlaser. Die Laser tastet das Höhenprofil der Schweißnaht ab und misst die Breite der Schweißnaht. Die Lasereinheit und der Prüfwagen sind mit einer Steuereinheit, d.h. einer Messstation gekoppelt. Die auf dem Prüfiniagen montierte Markiereinheit markiert die schadhaften Stellen der Schweißnaht.

Die erfindungsgemäße mobile Prüfvorrichtung und das erfindungsgemäße Prüfverfahren haben eine Reihe von Vorteilen gegenüber der herkömmlichen visuellen Prüfung.

Großrohre, wie sie für Offshore-Fundamente eingesetzt werden, haben einen Rohrdurchmesser von größer als 6 m, wobei Rohrdurchmesser von 8 m oder 10 m keine Seltenheit sind. Auch Rohrdurchmesser von z.B. 11,50 m oder 12 m sind möglich. Die Rundschweißnaht eines solchen Großrohrs sowohl an der Rohrinnenseite aber insbesondere an der Rohraußenseite hat daher eine erhebliche Länge.

Die zu prüfenden Rohre sind längsnaht- und rundnahtverschweißt Großrohre Längsnahtverschweißte Großrohrschüsse werden aus Stahlblechtafeln hergestellt. Der Blechtafelzuschnitt wird zunächst rund-gebogen und die offenen Kanten des so entstandenen geschlitzten Rohrschusses werden mit einer provisorischen Naht zusammengeheftet (Heftnaht), welche später in Produktionsprozess wieder entfernt wird. Der heftgeschweißte Rohrschuss wird an einem Schweißplatz positioniert und zunächst die Innennaht und dann die Außennaht am Rohrschuss angebracht. Anschließend kann das Rohr weiterbearbeitet, z.B. beschichtet werden. Die Rohre weisen bei üblicher Bauweise eine Wanddicke von 35 mm bis 250 mm, bevorzugt 40 bis 200 mm, besonders bevorzugt 50 mm bis 170 mm auf. Großrohre und Großrohrschüsse mit einem Durchmesser von 6 m bis 10 m haben bevorzugt eine Wanddicke von 50 mm bis 170 mm. Ein Großrohrschuss hat üblicherweise eine Länge von 2 m bis 4,2 m. Die Rohre werden aus einer Vielzahl von Rohrschüssen zusammengeschweißt und weisen dabei Längen von bis zu 120 m auf. Die Rohrschüsse werden über UP-Schweißen miteinander verbunden, wobei die zu prüfenden Schweißnähte erzeugt werden. Die Rohre wiegen in diesen Dimensionen im Mittel über tausend mehrere Tonnen.

Mit der Prüfvorrichtung ist eine sehr hohe Prüfgeschwindigkeit möglich. Der Prüfwagen fährt über das Rohr mit einer Geschwindigkeit von z.B. 70 mm/s. Dieses führt bei einem Rohr mit einem Rohrdurchmesser von 8 m zu einer Prüfzeit von nur noch 6 Minuten. Bei der visuellen Prüfung sind bisher 20 Minuten notwendig. Bei einem Rohrdurchmesser von 10 m kann eine Innenrundnaht mit der Prüfvorrichtung in ca. 8 min geprüft werden. Die visuelle Prüfung der Innennaht dauert im Vergleich mindestens 30 min.

Zudem verringert sich die notwendige Bewegung des Rohres. Zu Nahtprüfung, insbesondere der Außennahtprüfung, wird das Rohr üblicherweise auf einer Drehvorrichtung gelagert und um seine Längsachse gedreht. Während für die visuelle Prüfung zur Prüfung der Außen-Rund Schweißnaht und der Innenrohrnaht eine volle Drehung des Rohres notwendig ist, ist mit der Prüfvorrichtung gar keine oder nur noch eine halbe Drehung des Rohres notwendig. Das Rohr wird entgegen der Fahrtrichtung des Roboters gedreht, wenn die Außennaht vermessen wird, hierdurch ist weniger Drehung notwendig. Bei einer Prüfung der Außennaht bei einem Rohr in Relativbewegung, d.h. wenn der Prüfwagen entlang der Schweißnaht fährt und das Rohr gegenläufig gedreht wird, beträgt die Prüfdauer eines Rohrs mit 10 m Durchmesser ca. 15 min. Die visuelle Prüfung der Außennaht dauert im Vergleich mindestens 30 min.

Die Geschwindigkeit der Prüfung ist gegenüber der visuellen Prüfung somit deutlich erhöht. Der menschliche Einfluss bei der Schweißnahtprüfung und die subjektive Beurteilung wird durch die Verwendung der Prüfvorrichtung und das Verfahren eliminiert. Die Mitarbeiter werden entlastet werden, da die Aufgabe sehr ermüdend ist.

Wenn eine Echtzeitauswertung über eine Software erfolgt, hat dieses den Vorteil, dass die Markiereinheit direkt angesteuert werden kann und eine Kennzeichnung von Unregelmäßigkeiten direkt auf der Rohroberfläche erfolgen kann.

Wenn die erfindungsgemäße Prüfvorrichtung mit einer Speichereinheit ausgestattet ist oder mit einer Speichereinheit verbunden wird, können die von der Messeinheit gemessenen Daten als Bild oder Höhendatei gespeichert werden. Vorteil ist, dass alle Daten aufgezeichnet werden, einschließlich der markierten Schaltstellen und somit eine Dokumentation der Qualität und der Prüfung der Schweißnaht erstellt wird. Nach der Bearbeitung der als fehlerhaft markierten Stellen erfolgt eine neue Messung, um die Fehlerbehebung zu dokumentieren.

Die Datenaufzeichnung bietet eine Dokumentation aus der sich der genaue Zustand der Schweißnaht bei Übergabe des Rohrs zur weiteren Verwendung ergibt.

In einer weiteren Ausführungsform weist die Prüfvorrichtung zusätzlich zur Lasereinheit eine Wirbelstromprüfeinheit auf. Die Wirbelstromprüfeinheit ermöglicht eine Oberflächenriss-Prüfung. Hierfür fährt der Prüfwagen nach Vermessung der Schweißnaht mit der Lasereinheit in einem zweiten Lauf über die Nähte und nimmt die Wirbelstromprüfung vor. Hierdurch kann die Geschwindigkeit der Gesamtprüfung signifikant erhöht werden. Auch die Ergebnisse der Oberflächenrissprüfung können in diesem Fall aufgezeichnet und dokumentiert werden.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Seitenansicht einer Ausführungsform einer erfindungsgemäßen mobilen Prüfvorrichtung,
- Fig. 2: eine schematische Seitenansicht einer weitere Ausführungsform einer erfindungsgemä-ßen mobilen Prüfvorrichtung,
- Fig. 3: eine Frontansicht einer erfindungsgemäßen Prüfvorrichtung,
- Fig. 4: eine mobile Prüfvorrichtung bei der Messung an einem Großrohr und
- Fig. 5: ein schematischer Ablauf eines erfindungsgemäßen Verfahrens.

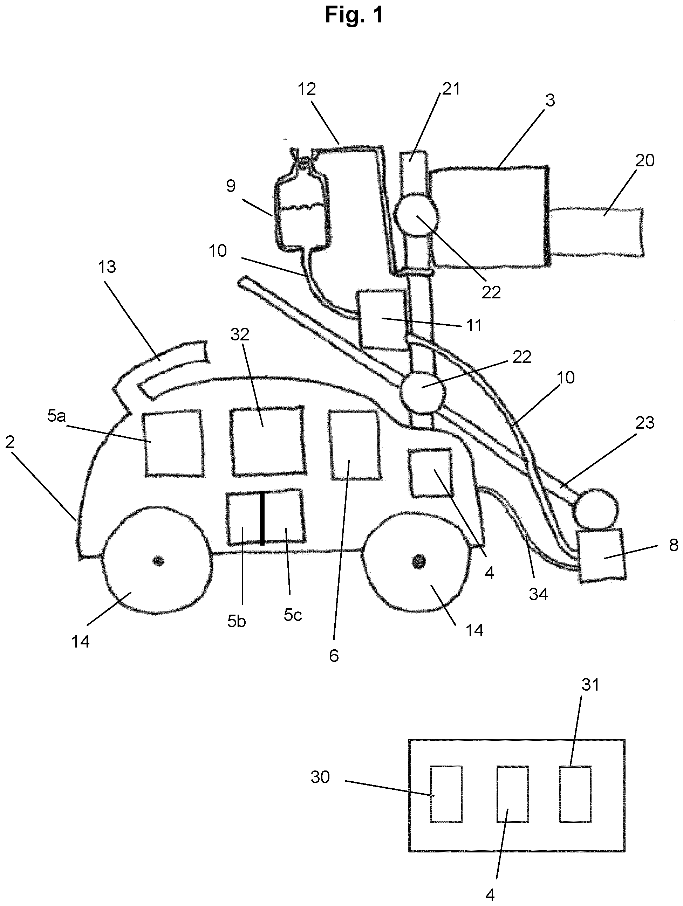

Figur 1 zeigt eine mobile Prüfvorrichtung in einer schematischen Seitenansicht. Die Prüfvorrichtung 1 zur Prüfung einer Rundschweißnaht eines Großrohrs weist einen verfahrbaren Prüfiniagen 2 als mobile Basiseinheit auf. In dieser Ausführungsform hat der Prüfwagen vier Räder 14, die jeweils paarweise an der Basiseinheit 2 montiert sind. Im Gehäuse des Prüfwagen sind eine Sende- und Empfangseinheit 4, eine Steuereinheit 5a für den Prüfwagen, eine Steuereinheit 5b für die Markiereinheit, eine Steuereinheit 5c für eine Messeinheit, eine Antriebseinheit 6 und ein Akkumulator 32 angeordnet. Die Antriebseinheit 6 treibt ein Paar der Räder 14 an. An einer Seite des Prüfwagens 2 ist eine Messeinheit 3 zur Prüfung der Schweißnaht erkennbar. Die Messeinheit 3 weist eine Lasereinheit mit einem Laserkopf 20 mit einem Emitter auf. Der Emitter kann mindestens einen Laserstrahl aussenden. Neben dem Laserkopf ist ein Detektor angeordnet (nicht dargestellt), der den reflektierten Laserstrahl auffängt. Die Messeinheit 3 ist an dem Prüfwagen mit einer Aufnahmevorrichtung 21 montiert. An der Aufnahmevorrichtung 21 befindet sich eine Einstellvorrichtung 22 mit der die Position der Messeinheit eingestellt werden kann, um ein optimales Messergebnis zu erzielen.

Auf der gleichen Seite am Prüfwagen wie die Messeinheit ist eine Markiereinheit 7 montiert, die mehrere Markierköpfe 8 aufweist, von denen nur einer erkennbar ist. Die Markierköpfe 8 sind über eine Farbleitung 10 mit einem Vorratsbehälter 9 für Farbe verbunden. Zwischen den Markierköpfen 8 und dem Vorratsbehälter 9 ist eine druckgesteuerte Schlauchpumpe 11 positioniert, die Farbe aus dem Vorratsbehälter 9 zum Markierkopf 8 pumpt. Der Vorratsbehälter 9 ist über eine Befestigung 12 an der Aufnahmevorrichtung 21 befestigt. Der Markierkopf 8 ist über eine Steuerleitung 34 mit der Steuereinheit 5b im Inneren des Prüfwagen verbunden. Die Markierköpfe sind über eine Halterung 23 an der Aufnahmevorrichtung 21 montiert. Über eine Einstellvorrichtung 22 kann ihre Position eingestellt werden.

Am Prüfwagen ist zudem ein Griff 13 angebracht mit dem der Prüfwagen angehoben und transportiert werden kann. Zusätzlich sind extern eine Auswerteeinheit 30 und eine Speichereinheit 31 vorgesehen, die Messdaten auswerten bzw. Messdaten oder Auswerteergebnisse speichern.

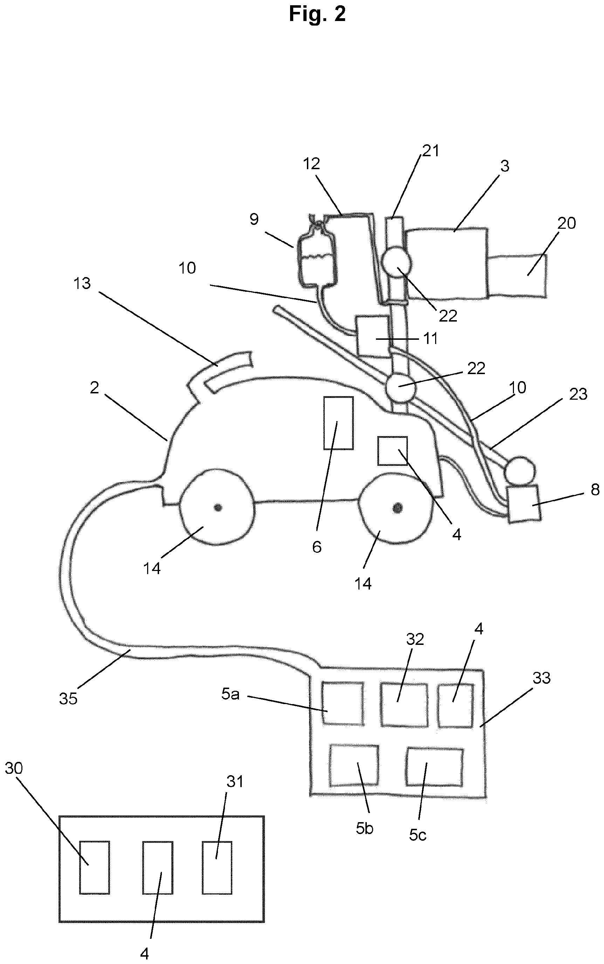

Figur 2 zeigt eine weitere Ausführungsform der erfindungsgemäßen Prüfvorrichtung 1. Bei dieser Ausführungsform sind mehrere Steuereinheiten 5 nicht im Prüfwagen, sondern extern angeordnet, z.B. in einem externen Steuermodul 33. Das externe Steuermodul 33 enthält eine Sende- und Empfangseinheit 4, eine Steuereinheit 5a für den Prüfwagen, eine Steuereinheit 5b für die Markiereinheit, eine Steuereinheit 5c für eine Messeinheit und einen Akkumulator 32. Über eine Versorgungsleitung 35 werden Energie und Signale zwischen Prüfwagen und Steuereinheit transportiert. Zusätzlich sind auch hier extern eine Auswerteeinheit 30 und eine Speichereinheit 31 vorgesehen, die Messdaten auswerten bzw. Messdaten oder Auswerteergebnisse speichern.

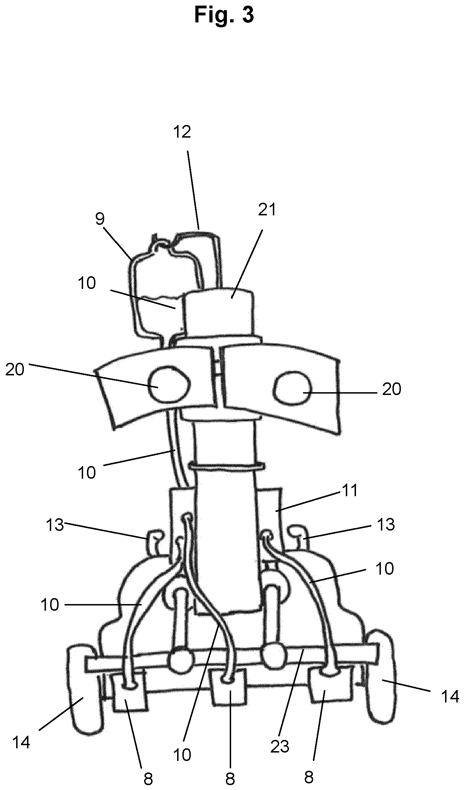

Figur 3 zeigt eine schematische Frontansicht einer Prüfvorrichtung. Die Messeinheit 3 ist an dem Prüfwagen 2 über eine Aufnahmevorrichtung 21 montiert. Die Messeinheit weist zwei nebeneinander angeordnete Laserköpfe 20 auf. Die Markiereinheit 7 hat drei nebeneinander angeordnete Markierköpfe 8. Die Markierköpfe 8 sind jeweils über eine Farbleitung 10 mit einer Pumpe 11 verbunden, die über eine weitere zentrale Farbleitung mit einem Vorratsbehälter 9 für Farbe verbunden ist. In der Messeinheit 3 sind zwei Laserköpfe 20 erkennbar, die nebeneinander angeordnet sind.

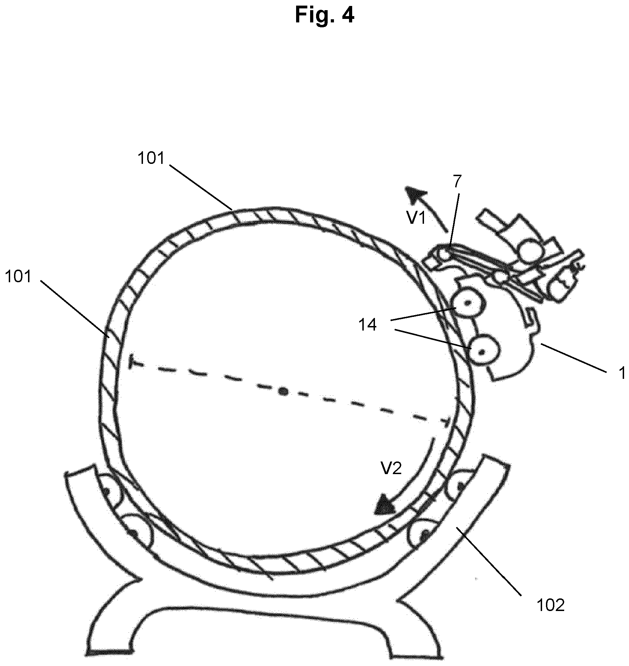

In Figur 4 ist eine mobile Prüfvorrichtung 1 bei der Messung an einem Großrohr 101 zu sehen. Die Räder 14 des Prüfwagens stehen auf der Rohroberfläche 101. Die Markiereinheit 7 ist mit geringem Abstand oberhalb der Rohrfläche angeordnet. Der Prüfwagen bewegt sich vorwärts in Richtung des Pfeils V1. Das Großrohr 100 liegt auf einer Drehvorrichtung 102, die das Großrohr in Richtung des Pfeils V2 drehen kann.

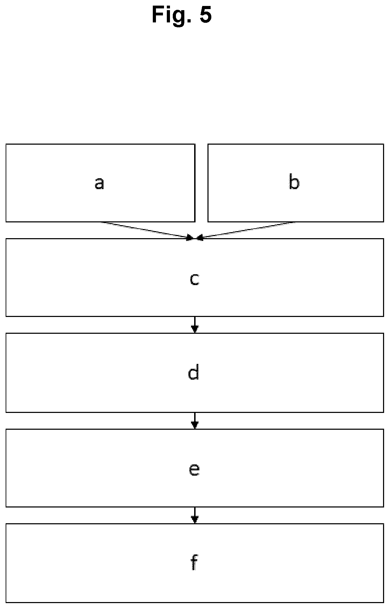

Figur 5 zeigt einen schematischen Ablauf eines erfindungsgemäßen Verfahrens zum Prüfen einer Rundschweißnaht an einem Großrohr. In Schritt a) wird ein Rohr mit Rundschweißnaht bereitgestellt ggf. auf einer Drehvorrichtung. Parallel wird in Schritt b) eine erfindungsgemäße Prüfvorrichtung bereitgestellt. Die Prüfvorrichtung wird in Schritt c) auf die Rohroberfläche im Bereich der Schweißnaht aufgesetzt. Im folgenden Schritt d) fährt der Prüfwagens der Prüfvorrichtung entlang der Schweißnaht und misst gleichzeitig die Beschaffenheit der Schweißnaht mit der Messeinheit durch Aussenden eines Laserstrahls mit mindestens einem Emitter und Auffangen des reflektierten Strahls mit einem Detektor. Gemäß Schritt e) werden die erhaltenen Messwerte (Istwerte) an eine Steuereinheit gesendet und ein Messwert wird mit einem Sollwert verglichen. Bei Feststellung einer Abweichung eines Messwerts vom Sollwert wird in Schritt f) ein Signal an eine Markiereinheit gesendet, die Markiereinheit aktiviert und die Rohroberfläche durch die Markiereinheit markiert.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

| | |
|---|---|
| Mobile Prüfvorrichtung | 1 |
| Prüfiniagen/Basiseinheit | 2 |
| Messeinheit | 3 |
| Sende- und Empfangseinheit | 4 |
| Steuereinheit | 5 |
| Antriebseinheit | 6 |
| Markiereinheit | 7 |
| Markierkopf | 8 |
| Vorratsbehälter für Farbe | 9 |
| Farbleitung | 10 |
| Schlauchpumpe | 11 |
| Befestigung | 12 |
| Griff | 13 |
| Griff | 13 |
| Rad | 14 |
| Laserkopf | 20 |
| Aufnahmevorrichtung | 21 |
| Einstellvorrichtung | 22 |
| Auswerteeinheit | 30 |
| Speichereinheit | 31 |
| Akkumulator | 32 |
| Steuermodul | 33 |
| Steuerleitung | 34 |
| Versorgungsleitung | 35 |
| Großrohr | 100 |
| Rohroberfläche | 101 |
| Drehvorrichtung | 102 |
| Pfeil Bewegungsrichtung Prüfwagen | V1 |
| Pfeil Bewegungsrichtung Rohr | V2 |

## Patentansprüche

1. Mobile Prüfvorrichtung (1) zur Prüfung einer Rundschweißnaht eines Großrohrs (100) aufweisend
- einen verfahrbaren Prüfwagen (2) als mobile Basiseinheit, aufweisend mindestens drei Räder (14), die an der Basiseinheit montiert sind,
- mindestens eine Messeinheit (3) zur Prüfung der Schweißnaht,
- mindestens eine Sende- und Empfangseinheit (4),
- mindestens eine Steuereinheit (5), und
- ggf. eine Antriebseinheit (6),
wobei die Messeinheit (3), ggf. die Sende- und Empfangseinheit (4) und ggf. die Steuereinheit (5) an der mobilen Basiseinheit (2) befestigt sind, **dadurch gekennzeichnet, dass**
- die Messeinheit (2) eine Lasereinheit ist und dass
- die Prüfvorrichtung ggf. mindestens eine Markiereinheit (7) aufweist, die an der mobilen Basiseinheit (2) befestigt ist.

2. Prüfvorrichtung (1) gemäß Patentanspruch 1, **dadurch gekennzeichnet, dass** die Messeinheit (2) eine Lasereinheit ist, die mindestens einen Laserkopf (20) mit einem Emitter hat, der mindestens einen Laserstrahl aussendet, und die mindestens einen Detektor hat, der den reflektierten Laserstrahl auffängt, wobei die Lasereinheit (2) bevorzugt eine Lichtlasereinheit ist.

3. Prüfvorrichtung (1) gemäß Patentanspruch 2, **dadurch gekennzeichnet, dass** die Messeinheit (2) über eine Aufnahmevorrichtung (21) mit einer Einstellvorrichtung (22) an der Basiseinheit befestigt ist und mit der Einstellvorrichtung (22) der Abstand zwischen Rohroberfläche (101) und Laser eingestellt ist.

4. Prüfvorrichtung (1) gemäß einem der vorhergehenden Patentansprüche, **dadurch gekennzeichnet, dass** der Prüfwagen (2) eine Lenkeinheit aufweist, die Lenkeinheit mit den Rädern (14) verbunden ist und die Lenkeinheit die Räder (14) lenkt.

5. Prüfvorrichtung (1) gemäß einem der vorhergehenden Patentansprüche, **dadurch gekennzeichnet, dass** die Steuereinheit (5) als Chip oder Softwareeinheit ausgebildet ist und die Steuereinheit (5) in einem Gerätegehäuse mit einem Filtersystem gekapselt ist und bevorzugt das Gehäuseinnere mit einem Überdruck beaufschlagt ist.

6. Prüfvorrichtung (1) gemäß einem der vorhergehenden Patentansprüche, **dadurch gekennzeichnet, dass** eine Markiereinheit (7) mindestens einen Markierkopf (8), mindestens einen Vorratsbehälter für Farbe (9) und eine Farbleitung (10) zwischen Markierkopf und Vorratsbehälter aufweist.

7. Prüfvorrichtung (1) gemäß Patentanspruch 6, **dadurch gekennzeichnet, dass** die Farbenköpfe (8) der Markiereinheit mit der Steuereinheit (5) verbunden sind und die Steuereinheit (5) die Markierköpfe (8) steuert.

8. Prüfvorrichtung (1) gemäß einem der Patentansprüche 6 oder 7, **dadurch gekennzeichnet, dass** die Markiereinheit eine druckgesteuerte Schlauchpumpe (11) aufweist, die Schlauchpumpe (11) mit dem Vorratsbehälter (9) verbunden ist und die Schlauchpumpe (11) die Farbe aus dem Vorratsbehälter (9) zum Markierkopf (8) pumpt, wobei die Schlaupumpe (11) bevorzugt einen Membrandruckschalter aufweist.

9. Prüfvorrichtung (1) gemäß einem der Patentansprüche 6 bis 8, **dadurch gekennzeichnet, dass** der Vorratsbehälter (9) eine Befestigung (12) mit Lager aufweist und mit der Befestigung auf der Basiseinheit drehbar gelagert ist.

10. Prüfvorrichtung (1) gemäß einem der vorhergehenden Patentansprüche, **dadurch gekennzeichnet, dass** die Räder (14) als magnetische Räder ausgebildet sind.

11. Prüfvorrichtung (1) gemäß einem der vorhergehenden Patentansprüche, **dadurch gekennzeichnet, dass** die Prüfvorrichtung eine Auswerteeinheit (30) aufweist, die die von der Messeinheit (2) erhaltenen Daten auswertet, und die Auswerteeinheit (30) bevorzugt eine Speichereinheit (31) aufweist, die von der Messeinheit (2) erhaltenen Daten und die von der Auswerteinheit (30) erzeugten Daten speichert.

12. Verfahren zum Prüfen einer Rundschweißnaht an einem Großrohr (100) umfassend die Schritte
a) Bereitstellen eines Rohrs (100) mit Rundschweißnaht ggf. auf einer Drehvorrichtung (102),
b) Bereitstellen einer Prüfvorrichtung (1) gemäß einer der Patentansprüche 1 bis 11,
c) Aufsetzen der Prüfvorrichtung (1) auf die Rohroberfläche (101) im Bereich der Schweißnaht,
d) Fahren des Prüfwagens der Prüfvorrichtung entlang der Schweißnaht und gleichzeitige Messen der Beschaffenheit der Schweißnaht mit einer Messeinheit (3) durch Aussenden eines Laserstrahls mit mindestens einem Emitter und Auffangen des reflektierten Strahls mit einem Detektor,
e) Senden der Messwerte (Istwerte) an eine Steuereinheit (5) und Vergleichen eines Messwerts mit einem Sollwert,
f) bei einer Abweichung eines Messwerts vom Sollwert, senden eines Signals an die Markiereinheit (3), aktivieren der Markiereinheit und Markieren der Rohroberfläche durch die Markiereinheit.

13. Verfahren gemäß Patentanspruch 12, **dadurch gekennzeichnet, dass** die in Schritt e) gesendeten Messwerte in einem weiteren Schritt an eine Speichereinheit gesendet und auf der Speichereinheit gespeichert werden.

14. Verfahren gemäß Patentanspruch 12 oder 13, **dadurch gekennzeichnet, dass** das Rohr in Schritt a) auf einer Drehvorrichtung bereitgestellt wird und dass während der Prüfiniagen gemäß Schritt d) entlang der Schweißnaht fährt, das Rohr um seine Längsachse gegenläufig zur Fahrtrichtung des Prüfwagens gedreht wird.

15. Verfahren gemäß einem der Patentansprüche 12 bis 14, **dadurch gekennzeichnet, dass** das Rohr einen Durchmesser von mindestens 6 m hat.
